# EUROPEAN PATENT SPECIFICATION

(11) **EP 1 542 741 B1**
(45) Date of publication and mention of the grant of the patent: **09.02.2011**
(21) Application number: 03770945.8
(22) Date of filing: 19.09.2003
(51) Int. Cl.: A61M 1/02, A61M 1/36

(54) **DEVICE AND METHOD FOR SEPARATING BLOOD INTO LEUKOCYTE DEPLETED BLOOD COMPONENTS**
VORRICHTUNG UND VERFAHREN ZUR SEPARIERUNG VON BLUT IN BLUTBESTANDTEILE MIT VERRINGERTEM LEUKOZYTENGEHALT
DISPOSITIF ET PROCEDE DE SEPARATION DE SANG EN COMPOSANTS SANGUINS APPAUVRIS EN LEUCOCYTES

(30) Priority: 20.09.2002 IT TO20020820
(43) Date of publication of application: 22.06.2005
(73) Proprietor: Fresenius Hemocare Italia S.r.l., I-41032 Cavezzo, (Modena) (IT)
(72) Inventor: MARI, Giorgio, I-41037 Mirandola (IT); VERRI, Paolo, I-41037 Mirandola (IT)
(74) Representative: Grund, Martin
(86) International application number: PCT/EP2003/010454
(87) International publication number: WO 2004/026372

(56) References cited:
- EP-A- 0 714 667
- WO-A-01/74158
- WO-A-03/063930

## Description

The present invention relates to a device and method for separating blood into leukocyte depleted blood components.

More particularly, the invention concerns a blood bag system used to separate blood components from whole blood and to remove leukocytes from the generated components.

US-4,596,657 describes a device of the above-mentioned type comprising a primary bag for collecting blood, e.g. from a donor, which is connected by means of a flexible conduit to a first satellite bag and through a second flexible conduit to a second satellite bag containing an additive solution; a leukocyte filter is arranged in the second flexible conduit which connects the primary bag to the second satellite bag.

The donor's whole blood collected in the primary bag is first centrifuged to separate the whole blood into a mixed layer of blood plasma and thrombocytes (platelet rich plasma or PRP) and an erythrocyte layer (packed red cells or PRC). The PRP layer is then fed into the first satellite bag and then the additive solution is fed from the second satellite bag into the primary bag. The PRC suspended in the additive solution are then fed from the primary bag, through the second conduit containing the leukocyte filter into the second satellite bag. The known device thus allows to obtain a leukocyte depleted PRC.

EP-A-0 879 608 describes a blood bag device which allows whole blood filtration and which also allows to obtain leukocyte depleted PRC. The described device comprises a collecting bag which is connected to a primary bag through a tubing containing a leukocyte filter and also through a by-pass line circumventing the filter. The donor's whole blood, collected in the collecting bag is first conveyed via the by-pass tubing into the primary container, wherein it is separated by centrifuging into a PRC layer and a PRP layer; the said PRP layer is then conveyed from the primary container into a first satellite container and subsequently, the PRC layer suspended in an additive agent is conveyed from the primary container via the tubing including the leukocyte filter into the collecting bag.

However, the removal of leukocytes from the other two blood components, namely platelet concentrate (PC) and plasma (PL) is also highly desirable.

In order to meet such a need, EP-A-0 556 303 discloses a blood bag system comprising a collecting bag and two satellite bags wherein each satellite bag is connected to the collecting bag through respective separate flexible conduits, each including a respective leukocyte filter. Whole blood is collected in the collecting bag and is centrifuged for separation into its component; then, the filtration of the individual blood component takes place in order to obtain leukocyte depleted PRP and PRC. The leukocyte depleted PRP may then be separated into PL and PC with the use of a third satellite bag.

While the solution of EP-A-0 556 303 is quite simple, such a solution is not cost-efficient nor space-efficient due to the use of a second leukocyte filter; particularly, difficulties may be encountered to fit into the centrifuge bucket four bags and two filters.

EP-A-0 714 667 describes an apparatus and methods of collecting blood cells using a first container to collect and separate blood. A filtration system is provided that has a first, dual purpose, flow path that is initially used to convey plasma and platelets from the first container to a pair of transfer/storage containers. A second flow path leads to a temporary transfer container through a filtration device. The first flow path bypasses the filtration device.

WO01/74158 A also describes a blood processing system and methods to collect leukocyte reduced blood components, including plasma that is free or virtually free of cellular blood species, such as red blood cells, platelets and leukocytes.

An object of the present invention is to provide a blood bag device allowing to separate whole blood into leukocyte depleted PRC, PL and PC which is economical and relatively compact, i.e. with a reduced bulk with respect to known prior art devices.

A further object of the invention is to provide a device which allows the above-mentioned separation with the use of a single leukocyte filter.

In addition, the invention has the objective of providing a method allowing whole blood to be separated into leukocyte depleted blood components with the use of a single leukocyte filter.

These and other objects of the invention are achieved by means of a device and a method as defined in the appended claims.

According to the invention, said second satellite container is further connected to said collecting container through conduit means by-passing said filter and said flow control means are adapted to allow fluid flow from said second satellite container into the collecting container only through the conduit means by-passing the filter.

According to the invention, the second satellite container initially contains a blood additive for storing erythrocytes such as a SAG-M solution and preferably the device further includes a third satellite container which is connected, in fluid flow communication, to the first satellite container for receiving from said first satellite container a leukocyte depleted third blood component.

According to a further preferred embodiment of the invention, the blood bag device of the invention comprises sensor means for detecting a parameter representative of the presence of said second hemocomponent in the filtrate from the leukocyte filter and the flow control means are adapted to switch fluid flow communication from said collecting container to said first satellite container to fluid flow communication from the collecting container to the second satellite container when the sensor means detect the presence of said second hemocomponent.

Further characterising features of the device and of the method for using the device for providing leukocyte depleted blood components are defined in the appended claims.

The invention will be further described in the following with reference to the annexed drawing which provides, by way of non limiting example, a schematic representation of the device of the invention in a specific embodiment.

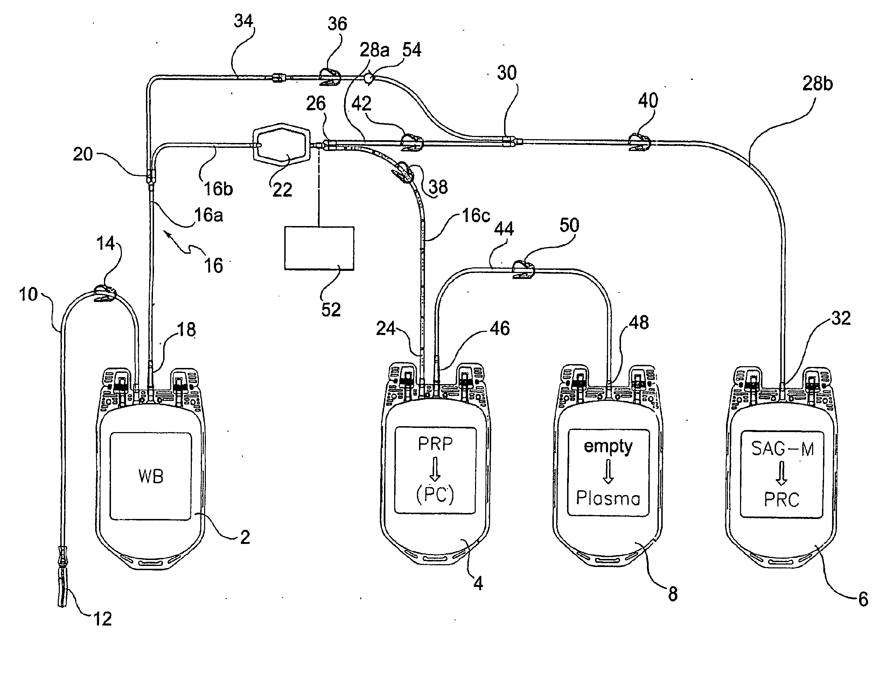

As shown in the drawing, the device for separating blood into blood components comprises a collecting container 2, a first satellite container 4, a second satellite container 6 and preferably a third satellite container 8 which, as known in the art, consist of flexible and pliable plastics bags.

Collecting bag 2, which is adapted to receive whole blood WB, may have an associated flexible tubing 10 with a hose clamp 14 and a terminal needle 12 adapted to be inserted into the donor or other whole blood supply source.

Collecting bag 2 is connected to first satellite bag 4 through a flexible tubing 16, including tubing section 16a extending from an outlet port 18 of collecting bag 2 to a 3-way connector 20, tubing section 16b extending from 3-way connector 20 to an inlet port of a leukocyte filter 22 and a tubing section 16c extending from an outlet port of leukocyte filter 22 to an inlet port 24 of first satellite bag 4.

Leukocyte filter 22, which is adapted for the depletion of the leukocyte content of blood components fed from collecting bag 2 to satellite bags 4 and 6, as it will be seen in more detail in the following, may consist of any leukocyte filter device known in the art such as described e.g. in EP-A-0 313 348, US-4,963,260 and US-5,580,465.

Said filter device 22 may include in addition to porous elements, specifically adapted for leukocyte removal, additional filter elements for the removal of gels and micro-aggregates.

Downstream of leukocyte filter 22, tubing section 16c has a branch off-provided by means of a 3-way connector 26, whereby the exit from the leukocyte filter is further connected to satellite bag 6 through tubing sections 28a, extending from tubing connector 26 to a 3-way tubing connector 30 and tubing section 28b extending from tubing connector 30 to an inlet port 32 of satellite bag 6.

Collecting bag 2 is further connected to satellite bag 6 through a by-pass tubing section 34, by-passing leukocyte filter 22 and extending from 3-way tubing connector 20 to 3-way tubing connector 30.

Valve means, typically consisting of hose clamps, identified with reference numerals 36, 38 and optionally 40 are provided, respectively on tubing sections 34, 16c and 28b; optionally, valve means consisting of a hose clamp 42 may also be provided on tubing section 28a.

The said valve means are adapted to cut off flow in the corresponding tubing sections and optionally can be automatically operated.

Preferably, in substitution or in addition to valve means 36, a one-way valve 54 is provided in tubing section 34 allowing fluid flow only from satellite bag 6 to collecting bag 2, to prevent unfiltered blood to flow into satellite bag 6.

The first satellite bag 4 is connected to third satellite bag 8 by means of a tubing 44 extending from an outlet port 46 of satellite bag 4 to an inlet port 48 of third satellite bag 8; valve means 50 can be provided to cut off flow in tubing 44.

The method for separating blood into leukocyte depleted blood components, with the use of the device of the invention according to the preferred embodiment hereinbefore described is as follows.

Whole blood WB is initially collected into collecting bag 2, which may initially contain an anti-coagulation agent. The whole blood in collecting bag 2 is then separated, in a known manner, e.g. by centrifuging into platelet rich plasma (PRP) and packed red cells (PRC).

The valve means, as above described, are then operated to allow fluid flow from collecting bag 2 to satellite bag 4 through leukocyte filter 22, while excluding fluid flow through by-pass tubing 34 and through tubings 28a and 28b; PRP is then transferred, e.g. by gravity, from collecting bag 2 into satellite bag 4 passing through leukocyte filter 22 to provide leukocyte depleted PRP into satellite bag 4 which is initially empty.

The valve means can then be selectively operated to allow fluid flow from satellite bag 6 into collecting bag 2 only through by-pass tubing 34, thereby to transfer an additive solution (e.g. SAG-M) from satellite bag 6 into collecting bag 2 containing the PRC.

The valve means are then operated to allow fluid flow communication from collecting bag 2 into satellite bag 6 through leukocyte filter 22, while excluding fluid flow through by-pass tubing 34, thereby to transfer PRC suspended in the additive solution from collecting bag 2 into satellite bag 6 under filtration conditions which provides for leukocyte depletion.

At the beginning of this second filtration step, filter 22 is still loaded with the PRP from the first filtration step. In order to allow recovery of the PRP filter hold-up into satellite bag 4, at the beginning of the second filtration step, preferably, valve means 38 are open to allow fluid flow into satellite bag 4, while valve means 42 and/or 40 are closed so as to prevent fluid flow into satellite bag 6; valve means 38 are then switched into the closed position and simultaneously, valve means 42 and 40 are switched into the open position as soon as the presence of red cells (PRC) is detected in the filtrate from filter 22.

To this end, sensor means (52) may optionally be provided for detecting a parameter which is indicative of the presence of red cells in the filtrate.

Sensor means suitable to detect the presence of red cells are known in the art and may consist, by way of example, of a colorimetric sensor device.

PRP collected in satellite bag 4 and already depleted from leukocytes can be centrifuged again, in a high spin, to separate plasma (PL) from platelet concentrate (PC) and the PL can be transferred into a third satellite bag 8 through tubing 44. Thus, the described method of use is able to generate three hemocomponents (PRC, PL and PC) all depleted of leukocytes, with the use of a single filter.

The invention has been herein described with reference to the simplest embodiment, wherein the flow control means are manually operated valves, such as hose clamps.

In a further and preferred embodiment of the invention, the (preferably disposable) blood bag equipment - as described - can be used in association with a (non-disposable) bag separator equipment, such as those conventionally used to squeeze the bags after centrifugation and separate the hemocomponents.

Such equipment shall include sensor means for detecting fluid flow or the presence of fluid at selected locations of the bags and/or conduit means and electro-mechanical valve means which are remotely controlled by the sensor means to provide fluid flow connection between the bags, according to the hereinbefore described process steps.

Preferably, the sensor means include a sensor which can detect red cells in the filtrate from leukocyte filter 22 and the control means are adapted to switch fluid flow communication in order to cut flow through tubing 16c and allow flow through tubings 28a and 28b when the presence of red cells is detected.

## Claims

1. A device for separating blood into blood components comprising:
- a collecting container (2) for receiving blood (WB),
- a first satellite container (4) connected, in fluid flow communication, to said collecting container (2) through a leukocyte filter (22) for receiving from said collecting container (2) a leukocyte depleted first blood component,
- a second satellite container (6) connected, in fluid flow communication, with said collecting container (2) for receiving from said collecting container a second leukocyte depleted blood component, wherein
said second satellite container (6) is connected to said collecting container (2) through said leukocyte filter (22), said leukocyte filter being adapted to allow said first satellite container (4) to receive from said collecting container a leukocyte depleted platelet rich plasma component and to allow said second satellite container to receive from said collecting container a leukocyte depleted packed red cells component, flow control means (36, 38, 42) being provided for allowing fluid flow from said collecting container selectively into said first (4) or second (6) satellite container through said leukocyte filter (22), whereby whole blood (WB) can be separated into a leukocyte depleted platelet rich plasma component and a leukocyte depleted packed red cells component (PRC) with a single leukocyte filter (22), wherein said second satellite container (6) includes a blood additive and is further connected to said collecting container (2) through conduit means (28b, 34, 16a) by-passing said filter (22) and wherein the said flow control means (36, 38, 42) is further adapted for allowing fluid flow from said second satellite container (6) into said collecting container (2) only through said conduit means (28b, 34, 16a) by-passing said filter (22), thereby to allow feeding said blood additive from said second satellite container (6) into said collecting container (12) through said conduit means (28b 34, 16a) by-passing said filter.

2. A device according to claim 1, **characterised in that** said flow control means (36, 38, 42) are adapted to selectively:
- feeding a platelet rich plasma component (PRP) from said collecting container (2) into said first satellite container (4) through said leukocyte filter (22) to provide a leukocyte depleted platelet rich plasma component into said first satellite container (4);
- feeding said blood additive from said second satellite container (6) into said collecting container (2) only through said conduit means (28, 34, 16a) by-passing said filter (22); and
- feeding a packed red cells component (PRC) from said collecting container (2) into said second satellite container (6) only through said leukocyte filter (22) to provide into said second satellite container (6) a leukocyte depleted packed red cells component (PRC).

3. A device according to any of the preceding claims, **characterised in that** it comprises:
- first conduit means (16a, 16b, 16c) connecting said collecting container (2) to said first satellite container (4) through said leukocyte filter (22),
- second conduit means (28a, 28b) branching off (26) from said first conduit means (16c) downstream of said leukocyte filter (22), thereby to connect said collecting container (2) to said second satellite container (6), and
- by-pass conduit means (34) branching off (20) from said first conduit means (16a), upstream of said leukocyte filter (22) and connected to said second conduit means (28b).

4. A device according to any of claims 1 to 3, **characterised in that** said flow control means (36, 38, 42) comprise sensor means for detecting fluid flow or presence of fluid at selected positions of the device and electro-mechanical valve means (36, 38, 42) operated and controlled by said sensor means.

5. A device according to any of claims 1 to 4, **characterised in that** said flow control means comprise sensor means (52) for detecting a parameter representative of the presence of said packed red cells component (PRC) in the filtrate from said leukocyte filter (22) and automatically operated valve means (38, 42) adapted to switch fluid flow communication from said collecting container (2) to said first satellite container (4) to fluid flow communication from said collecting container (2) to said second satellite container (6) when the sensor means (52) detect the presence of said packed red cells component (PRC).

6. A device according to any of claims 1 to 3, **characterised in that** said flow control means (36, 38, 42) comprise manually operated valves.

7. A device according to claim 3, **characterised in that** a one-way valve (54 or 36) is provided in by-pass conduit means (34) allowing fluid flow only from second satellite container (6) to collecting container (2).

8. A device according to claim 3, **characterised in that** said flow control means comprise valve means (40, 42) in said second conduit means (28a, 28b).

9. A device according to any of claims 1 to 4, **characterised in that** said flow control means (36, 38, 40, 42, 52) are associated with a separator device adapted to cause fluid flow from the collecting container (2) to the satellite containers (4, 6).

10. A device according to any of claims 1 to 9, **characterised in that** it further comprises a third satellite container (8) connected in fluid flow communication with said first satellite container (4) for receiving from said first satellite container (4) a plasma component (PL).

11. A method for separating blood into leukocyte depleted blood components comprising the steps of:
- providing a blood separator device comprising a collecting container (2) for receiving blood, a first satellite container (4) connected, in fluid flow communication, to said collecting container (2) through a leukocyte filter (22) and a second satellite container (6) connected, in fluid flow communication, to said collecting container through said leukocyte filter (22), said second satellite container including a blood additive,
- separating blood collected in said collecting container (2) into a platelet rich plasma component (PRP) and a packed red cells component (PRC),
- feeding said platelet rich plasma component from said collecting container (2) into said first satellite container (4) through said leukocyte filter (22) to provide a leukocyte depleted platelet rich plasma component into said first satellite container (4), while leaving the packed red cells component within said collecting container (2),
- adding into said collecting container (2) the additive solution for the packed red cells component (PRC), by feeding said additive solution from said second satellite container (6) into said collecting container (2) through by-pass conduit means (34), by-passing said leukocyte filter (22),
- feeding said packed red cells component (PRC) suspended in said additive into said second satellite container (6) passing through said leukocyte filter (22).

12. A method according to claim 11, comprising the steps of:
- detecting the presence of said packed red cells component (PRC) in the filtrate from said leukocyte filter (22) and
- switching fluid flow communication from said collecting container (2) to said first satellite container (4) to fluid flow communication from said collecting container (2) to said second satellite container (6) when the presence of said packed red cells component is detected in the filtrate, thereby to allow recovery into said first satellite container (4) of the filter hold-up of the platelet rich plasma component (PRP).

13. A method according to claims 11 to 12, further comprising separating the leukocyte depleted platelet rich plasma component (PRP) in said first satellite container (4) into a plasma (PL) component and a platelet concentrate (PC) component and feeding said plasma component (PL) from said first satellite container (4) into a third satellite container (8).

14. A method according to any of claims 11 to 13, carried out with the use of a device according to any of claims 1 to 10.

## Patentansprüche

1. Vorrichtung zur Trennung von Blut in Blutkomponenten, umfassend:
- einen Sammelbehälter (2) zum Empfangen von Blut (WB),
- einen ersten Satellitenbehälter (4), der in Flüssigkeitsflussverbindung durch einen Leukozytenfilter (22) mit dem Sammelbehälter (2) verbunden ist, um von dem Sammelbehälter (2) eine leukozytendepletierte erste Blutkomponente zu empfangen,
- einen zweiten Satellitenbehälter (6), der in Flüssigkeitsflussverbindung mit dem Sammelbehälter (2) verbunden ist, um von dem Sammelbehälter (2) eine leukozytendepletierte zweite Blutkomponente zu empfangen, wobei der zweite Satellitenbehälter (6) mit dem Sammelbehälter (2) durch den Leukozytenfilter (22) verbunden ist, wobei der Leukozytenfilter angepasst ist, um es dem ersten Satellitenbehälter (4) zu ermöglichen, von dem Sammelbehälter eine leukozytendepletierte, plättchenreiche Plasmakomponente zu empfangen, und um es dem zweiten Satellitenbehälter zu ermöglichen, von dem Sammelbehälter eine leukozytendepletierte Komponente gepackter roter Blutkörperchen zu empfangen, wobei Flusskontrollmittel (36, 38, 42) zur Verfügung gestellt werden, um einen Flüssigkeitsfluss selektiv von dem Sammelbehälter in den ersten (4) oder zweiten (6) Satellitenbehälter durch den Leukozytenfilter (22) zu ermöglichen, wodurch Vollblut (WB) mit einem einzigen Leukozytenfilter (22) in eine leukozytendepletierte plättchenreiche Plasmakomponente und eine leukozytendepletierte gepackte rote Blutzellenkomponente (PRC) getrennt werden kann, wobei der zweite Satellitenbehälter (6) einen Blutzusatz einschließt, und weiter durch Leitungen (28b, 34, 16a), die den Filter (22) umgehen, mit dem Sammelbehälter (2) verbunden ist, und wobei das Flusskontrollmittel (36, 38, 42) weiter angepasst ist, um einen Flüssigkeitsfluss von dem zweiten Satellitenbehälter (6) in den Sammelbehälter (2) nur durch die Leitungen (28b, 34, 16a), die den Filter umgehen, zu ermöglichen, um **dadurch** das Zuführen des Blutzusatzes von dem zweiten Satellitencontainer (6) in den Sammelbehälter (12) durch die Leitungen (28b, 34, 16a), die den Filter umgehen, zu ermöglichen.

2. Vorrichtung gemäß Anspruch 1, **dadurch gekennzeichnet, dass** die Flusskontrollmittel (36, 38, 42) angepasst sind, um selektiv:
- eine plättchenreiche Plasmakomponente (PRP) von dem Sammelbehälter (2) durch den Leukozytenfilter (22) in den ersten Satellitenbehälter (4) zuzuführen, um eine leukozytendepletierte plättchenreiche Plasmakomponente in den ersten Satellitenbehälter (4) zu liefern;
- den Blutzusatz von dem zweiten Satellitenbehälter (6) in den Sammelbehälter (2) nur durch die Leitungen (28, 34, 16a), die den Filter (22) umgehen, zuzuführen; und
- eine gepackte rote Blutzellenkomponente (PRC) von dem Sammelbehälter (2) in den zweiten Satellitenbehälter (6) nur durch den Leukozytenfilter (22) zuzuführen, um in den zweiten Satellitenbehälter (6) eine leukozytendepletierte gepackte rote Blutzellenkomponente (PRC) zu liefern.

3. Vorrichtung gemäß einem der vorstehenden Ansprüche, **dadurch gekennzeichnet, dass** sie folgendes umfasst:
- erste Leitungen (16a, 16b, 16c), die den Sammelbehälter (2) durch den Leukozytenfilter (22) mit dem ersten Satellitenbehälter (4) verbinden,
- zweite Leitungen (28a, 28b), die von der ersten Leitung (16c) unterhalb des Leukozytenfilters (22) abzweigen (26), um **dadurch** den Sammelbehälter (2) mit dem zweiten Satellitenbehälter (6) zu verbinden, und
- eine Umgehungsleitung (34), die von der ersten Leitung (16a) oberhalb des Leukozytenfilters (22) abzweigt (20), und die mit der zweiten Leitung (28b) verbunden ist.

4. Vorrichtung gemäß einem der Ansprüche 1-3, **dadurch gekennzeichnet, dass** die Flusskontrollmittel (36, 38, 42) Sensoren umfassen, um einen Flüssigkeitsfluss oder die Anwesenheit von Flüssigkeit an ausgewählten Positionen der Vorrichtung zu detektieren, und elektromechanische Ventile (36, 38, 42), die von den Sensoreinrichtungen betrieben und kontrolliert werden.

5. Vorrichtung gemäß einem der Ansprüche 1 bis 4, **dadurch gekennzeichnet, dass** die Flusskontrollmittel einen Sensor (52) zur Detektion eines für die Anwesenheit der gepackten roten Blutzellkomponente (PRC) charakteristischen Parameters in dem Filtrat des Leukozytenfilters (22) umfassen und automatisch betriebene Ventile (38, 42), die angepasst sind, um die Flüssigkeitsflussverbindung von dem Sammelbehälter (2) zu dem ersten Satellitenbehälter (4) zu einer Flüssigkeitsflussverbindung von dem Sammelbehälter (2) zu dem zweiten Satellitenbehälter (6) umzustellen, wenn der Sensor (52) die Anwesenheit der gepackten roten Blutzellkomponente (PRC) detektiert.

6. Vorrichtung gemäß einem der Ansprüche 1 bis 3, **dadurch gekennzeichnet, dass** die Flusskontrollmittel (36, 38, 42) manuell betriebene Ventile umfassen.

7. Vorrichtung gemäß Anspruch 3, **dadurch gekennzeichnet, dass** ein Einwegventil (54 oder 36), das einen Flüssigkeitsfluss nur vom zweiten Satellitenbehälter (6) zum Sammelbehälter (2) erlaubt, in der Umgehungsleitung (34) zur Verfügung gestellt wird.

8. Vorrichtung gemäß Anspruch 3, **dadurch gekennzeichnet, dass** die Flusskontrollmittel Ventile (40, 42) in den zweiten Leitungen (28a, 28b) umfassen.

9. Vorrichtung gemäß einem der Ansprüche 1 bis 4, **dadurch gekennzeichnet, dass** die Flusskontrollmittel (36, 38, 40, 42, 52) mit einer Trennungsvorrichtung verbunden sind, die angepasst ist, um einen Flüssigkeitsfluss von dem Sammelbehälter (2) in die Satellitenbehälter (4, 6) zu verursachen.

10. Vorrichtung gemäß einem der Ansprüche 1 bis 9, **dadurch gekennzeichnet, dass** sie weiter einer dritten Satellitenbehälter (8) umfasst, der in Flüssigkeitsflussverbindung mit dem ersten Satellitenbehälter (4) verbunden ist, um von dem ersten Satellitenbehälter (4) eine Plasmakomponente (PL) zu erhalten.

11. Verfahren zur Trennung von Blut in leukozytendepletierte Blutkomponenten, umfassend die Schritte:
- zur Verfügung stellen einer Bluttrennungsvorrichtung, die einen Sammelbehälter (2) zum Empfangen von Blut, einen ersten Satellitenbehälter (4), der in Flüssigkeitsflussverbindung durch einen Leukozytenfilter (22) mit dem Sammelbehälter (2) verbunden ist umfasst, und einen zweiten Satellitenbehälter (6), der in Flüssigkeitsflussverbindung mit dem Sammelbehälter durch den Leukozytenfilter (22) verbunden ist, wobei der zweite Satellitenbehälter einen Blutzusatz einschließt,
- Trennen des in dem Sammelbehälter (2) gesammelten Bluts in eine plättchenreiche Komponente (PRP) und eine Komponente aus gepackten roten Blutzellen (PRC)
- Zuführen der plättchenreichen Komponente vom Sammelbehälter (2) durch den Leukozytenfilter (22) in den ersten Satellitenbehälter (4), wobei die Komponente aus gepackten roten Blutzellen im Sammelbehälter (2) belassen wird,
- Zugeben der Zusatzlösung für die Komponente aus gepackten roten Blutzellen (PRC) in den Sammelbehälter (2) durch Zuführen der Zusatzlösung vom zweiten Satellitenbehälter (6) in den Sammelbehälter (2) durch die Umgehungsleitung (34), die den Leukozytenfilter (22) umgeht,
- Zuführen der gepackten roten Blutzellkomponente (PRC), die in dem Zusatz suspendiert ist, durch den Leukozytenfilter (22) in den zweiten Satellitenbehälter (6).

12. Verfahren gemäß Anspruch 11, umfassend die Schritte:
- Detektieren der Anwesenheit der gepackten roten Blutzellkomponente (PRC) in dem Filtrat des Leukozytenfilters (22) und
- Umstellen der Flüssigkeitsflussverbindung von dem Sammelbehälter (2) zu dem ersten Satellitenbehälter (4) zu einer Flüssigkeitsflussverbindung von dem Sammelbehälter (2) zu dem zweiten Satellitenbehälter (6), wenn die Anwesenheit der gepackten roten Blutzellkomponente in dem Filtrat detektiert wird, um **dadurch** das Auffangen des Filterrückhalts der plättchenreichen Plasmakomponente (PRP) in dem ersten Satellitenbehälter (4) zu ermöglichen.

13. Verfahren gemäß den Ansprüchen 11 bis 12, weiter umfassend das Trennen der leukozytendepletierten plättchenreichen Komponente (PRP) in dem ersten Satellitenbehälter (4) in eine Plasmakomponente (PL) und eine Plättchenkonzentratkomponente (PC), und das Zuführen der Plasmakomponente (PL) von dem ersten Satellitenbehälter (4) in einen dritten Satellitenbehälter (8).

14. Verfahren gemäß einem der Ansprüche 11 bis 13, ausgeführt unter Verwendung einer Vorrichtung gemäß einem der Ansprüche 1 bis 10.

## Revendications

1. Dispositif de séparation de sang en composants sanguins, comprenant :
- un conteneur de recueil (2) pour recevoir du sang complet (WB)
- un premier conteneur satellite (4) raccordé, en communication de fluide, audit conteneur de recueil (2) par un filtre de leucocytes (22) pour recevoir dudit conteneur de recueil (2) un premier composant sanguin appauvri en leucocytes,
- un deuxième conteneur satellite (6) raccordé, en communication de fluide, avec ledit conteneur de recueil (2) pour recevoir dudit conteneur de recueil, un deuxième composant sanguin appauvri en leucocytes, dans lequel
ledit deuxième conteneur satellite (6) est raccordé audit conteneur de recueil (2) par ledit filtre de leucocytes (22), ledit filtre de leucocytes étant adapté pour permettre audit premier conteneur satellite (4) de recevoir dudit conteneur de recueil, un composant plasmatique riche en plaquettes et appauvri en leucocytes et pour permettre audit deuxième conteneur satellite de recevoir dudit conteneur de recueil, un composant d'hématies concentrées appauvri en leucocytes, des moyens de contrôle de l'écoulement (36, 38, 42) étant aménagés pour permettre l'écoulement de fluide dudit conteneur de recueil, sélectivement, dans lesdits premier (4) ou deuxième (6) conteneurs satellites par ledit filtre de leucocytes (22), moyennant quoi le sang complet (WB) peut être séparé en un composant plasmatique riche en plaquettes et appauvri en leucocytes et un composant d'hématies concentrées (PRC) appauvri en leucocytes avec un filtre de leucocytes unique (22) dans lequel ledit deuxième conteneur satellite (6) comprend un additif sanguin et est en outre raccordé audit conteneur de recueil (2) par des moyens de conduite (28b, 34, 16a) en contournant ledit filtre (22) et dans lequel lesdits moyens de contrôle de l'écoulement (36, 38, 42) sont en outre adaptés pour permettre au fluide de s'écouler dudit deuxième conteneur satellite (6) dans ledit conteneur de recueil (2) uniquement par lesdits moyens de conduite (28b, 34, 16a) contournant ledit filtre (22), pour permettre ainsi l'apport dudit additif sanguin dudit deuxième conteneur satellite (6) dans ledit conteneur de recueil (12) par lesdits moyens de conduite (28b, 34, 16a) contournant ledit filtre.

2. Dispositif selon la revendication 1, **caractérisé en ce que** lesdits moyens de contrôle de l'écoulement (36, 38, 42) sont adaptés sélectivement pour :
- acheminer un composant plasmatique riche en plaquettes (PRP) dudit conteneur de recueil (2) dans ledit premier conteneur satellite (4) par ledit filtre de leucocytes (22) pour acheminer un composant plasmatique riche en plaquettes, appauvri en leucocytes dans ledit premier conteneur satellite (4) ;
- acheminer ledit additif sanguin dudit deuxième conteneur satellite (6) dans ledit conteneur de recueil (2) uniquement par lesdits moyens de conduite (28, 34, 16a) en contournant ledit filtre (22) ; et
- acheminer un composant d'hématies concentrées (PRC) dudit conteneur de recueil (2) dans ledit deuxième conteneur satellite (6) uniquement par ledit filtre de leucocytes (22) pour acheminer dans ledit deuxième conteneur satellite (6) un composant d'hématies concentrées appauvri en leucocytes (PRC).

3. Dispositif selon l'une quelconque des revendications précédentes, **caractérisé en ce qu'**il comprend :
- des premiers moyens de conduite (16a, 16b, 16c) raccordant ledit conteneur de recueil (2) audit premier conteneur satellite (4) par ledit filtre de leucocytes (22),
- des deuxièmes moyens de conduite (28a, 28b) bifurquant (26) desdits premiers moyens de conduite (16c) en aval dudit filtre de leucocytes (22), pour raccorder ainsi ledit conteneur de recueil (2) audit deuxième conteneur satellite (6), et
- des moyens de conduite de dérivation (34) bifurquant (20) desdits premiers moyens de conduite (16a) en amont dudit filtre de leucocytes (22) et raccordés auxdits deuxièmes moyens de conduite (28b).

4. Dispositif selon l'une quelconque des revendications 1 à 3, **caractérisé en ce que** lesdits moyens de contrôle de l'écoulement (36, 38, 42) comprennent des moyens de détection pour détecter l'écoulement de fluide ou la présence de fluide à des positions sélectionnées du dispositif et des moyens de valve électromagnétique (36, 38, 42) actionnés et contrôlés par lesdits moyens de détection.

5. Dispositif selon l'une quelconque des revendications 1 à 4, **caractérisé en ce que** lesdits moyens de contrôle de l'écoulement comprennent des moyens de détection (52) pour détecter un paramètre représentatif de la présence dudit composant d'hématies concentrées (PRC) dans le filtrat dudit filtre de leucocytes (22) et des moyens de valve (38, 42) actionnés automatiquement, adaptés pour passer, en communication de fluide, dudit conteneur de recueil (2) audit premier conteneur satellite (4), en communication de fluide, dudit conteneur de recueil (2) audit deuxième conteneur satellite (6) lorsque les moyens de détection (52) détectent la présence dudit composant d'hématies concentrées (PRC).

6. Dispositif selon l'une quelconque des revendications 1 à 3, **caractérisé en ce que** lesdits moyens de contrôle de l'écoulement (36, 38, 42) comprennent des valves actionnées manuellement.

7. Dispositif selon la revendication 3, **caractérisé en ce qu'**une valve unidirectionnelle (54 ou 36) est aménagée dans des moyens de conduite de dérivation (34) permettant au fluide de s'écouler uniquement du deuxième conteneur satellite (6) au conteneur de recueil (2).

8. Dispositif selon la revendication 3, **caractérisé en ce que** lesdits moyens de contrôle de l'écoulement comprennent des moyens de valves (40, 42) dans lesdits deuxièmes moyens de conduite (28a, 28b).

9. Dispositif selon l'une quelconque des revendications 1 à 4, **caractérisé en ce que** lesdits moyens de contrôle de l'écoulement (36, 38, 40, 42, 52) sont associés à un dispositif séparateur adapté pour entraîner l'écoulement du fluide du conteneur de recueil (2) vers les conteneurs satellites (4, 6).

10. Dispositif selon l'une quelconque des revendications 1 à 9, **caractérisé en ce qu'**il comprend en outre un troisième conteneur satellite (8) raccordé, en communication de fluide, avec ledit premier conteneur satellite (4) pour recevoir dudit premier conteneur satellite (4), un composant plasmatique (PL).

11. Procédé de séparation de sang en composants sanguins appauvris en leucocytes comprenant les étapes consistant à :
- fournir un dispositif séparateur sanguin comprenant un conteneur de recueil (2) pour recevoir du sang, un premier conteneur satellite (4) raccordé, en communication de fluide, audit conteneur de recueil (2) par un filtre de leucocytes (22) et un deuxième conteneur satellite (6) raccordé, en communication de fluide, audit conteneur de recueil par ledit filtre de leucocytes (22), ledit deuxième conteneur satellite comprenant un additif sanguin,
- séparer du sang recueilli dans ledit conteneur de recueil (2) dans un composant plasmatique riche en plaquettes (PRP) et un composant d'hématies concentrées (PRC),
- acheminer ledit composant plasmatique riche en plaquettes dudit conteneur de recueil (2) dans ledit premier conteneur satellite (4) par ledit filtre de leucocytes (22) pour fournir un composant plasmatique riche en plaquettes, appauvri en leucocytes dans ledit premier conteneur satellite (4) tout en laissant le composant d'hématies concentrées dans ledit conteneur de recueil (2),
- ajouter dans ledit conteneur de recueil (2) la solution d'additif pour le composant d'hématies concentrées (PRC) en acheminant ladite solution d'additif dudit deuxième conteneur satellite (6) dans ledit conteneur de recueil (2) par des moyens de conduite de dérivation (34), en contournant ledit filtre de leucocytes (22),
- acheminer ledit composant d'hématies concentrées (PRC) en suspension dans ledit additif, dans ledit deuxième conteneur satellite (6) en passant par ledit filtre de leucocytes (22).

12. Procédé selon la revendication 11, comprenant les étapes consistant à :
- détecter la présence dudit composant d'hématies concentrées (PRC) dans le filtrat dudit filtre de leucocytes (22) et
- passer, en communication de fluide, dudit conteneur de recueil (2) audit premier conteneur satellite (4) en communication de fluide dudit conteneur de recueil (2) audit deuxième conteneur satellite (6) lorsque la présence dudit composant d'hématies concentrées est détectée dans le filtrat, pour permettre ainsi la récupération dans ledit premier conteneur satellite (4) du rétentat de filtrage du composant plasmatique riche en plaquettes (PRP).

13. Procédé selon les revendications 11 à 12, comprenant en outre la séparation du composant plasmatique riche en plaquettes appauvri en leucocytes (PRP) dans ledit premier conteneur satellite (4) en un composant plasmatique (PL) et un composant de concentré plaquettaire (PC) et l'acheminement dudit composant plasmatique (PL) dudit premier conteneur satellite (4) dans un troisième conteneur satellite (8).

14. Procédé selon l'une quelconque des revendications 11 à 13, réalisé en utilisant un dispositif selon l'une quelconque des revendications 1 à 10.
